# EUROPEAN PATENT SPECIFICATION

(11) **EP 4 545 040 B1**
(45) Date of publication and mention of the grant of the patent: **01.07.2026**
(21) Application number: 24209103.1
(22) Date of filing: 28.10.2024
(51) Int. Cl.: A61B 90/35, A61B 17/00

(54) **SUPPORT ARM AND SURGICAL LIGHT**
TRAGARM UND OPERATIONSLEUCHTE
BRAS DE SUPPORT ET LAMPE CHIRURGICALE

(30) Priority: 27.10.2023 CN 202311415755
(43) Date of publication of application: 30.04.2025
(73) Proprietor: Nanjing Mindray Bio-Medical Electronics Co., Ltd., Nanjing, Jiangsu 211111 (CN)
(72) Inventor: SUN, Chao, Nanjing, 211111 (CN); XU, Xiaotian, Nanjing, 211111 (CN); HE, Qicheng, Nanjing, 211111 (CN); GUO, Bin, Nanjing, 211111 (CN)
(74) Representative: KIPA AB

(56) References cited:
- WO-A2-2014/145188
- US-A1- 2017 079 660
- US-A1- 2022 240 989

## Description

### TECHNICAL FIELD

The disclosure relates to the technical field of medical devices, and more particularly to a support arm and a surgical light.

### BACKGROUND

In related technologies, a surgical light in an operating room employs a support arm for connection. Wherein an insertion portion at a light head assembly is inserted into the support arm, and a securing element is inserted radially into the support arm and the insertion portion. Then, the support arm is sleeved inside a decorative cover which is fixed to the support arm with screws. However, it is possible to forget to arrange the screws after the decorative cover is sleeved. Then during a process of disinfecting, wiping or rotating the surgical light, the decorative cover is easily moved along an axial direction to expose the securing element. In such a way, the securing element has a high risk of disengagement, which results in serious medical accidents.

The WO 2014/145188 A2 relates to a surgical end effector of a surgical robotic manipulator. In particular, the end effector includes a nose tube and a cutting accessory that is removably engaged with the nose tube. It discloses an axial connector comprising a locking member and a lock collar.

### SUMMARY

The present invention is defined by the features of the independent claim. Embodiments of the invention are defined in the dependent claims.

In view of this, the embodiments of this disclosure are expected to provide a support arm and a surgical light, which is capable of reducing risk of disengagement for a securing element and improving safety of the support arm and the surgical light.

An embodiment of this disclosure provides a support arm, including:
an insertion portion, which is configured to connect a medical device;
an arm body, which is provided with a connector, wherein the connector is provided inside with an insertion cavity for inserting the insertion portion; wherein a side wall of the connector is provided with a clamping groove;
a securing element;
a ring that is capable of preventing the securing element from disengaging from the insertion portion, wherein the ring is provided with an insertion groove, and movably sleeved around an outer circumference of the connector, so as to at least move between a first position and a second position;
when the ring is at the first position, the insertion groove is connected with the clamping groove, so as to enable the securing element to be inserted into the clamping groove through the insertion groove to engage with the insertion portion, so as to further enable the insertion portion to be suspended on the securing element and to be capable of rotating relative to the connector; when the ring is at the second position, the ring at least partially covers the securing element, so as to prevent the securing element from disengaging from the insertion portion.

When assembling the support arm of this embodiment of this disclosure, the ring is sleeved around an outer circumference of the connector, the insertion portion is inserted into the insertion cavity, the ring is moved to the first position, so as to align the insertion groove with the clamping groove, and then the securing element is inserted into the clamping groove through the insertion groove, and further inserted into the insertion portion. That is to say, a portion of the securing element is located inside the insertion portion and another portion of the securing element is located inside the clamping groove. Then, the ring is moved, so as to least partially misaligned the insertion groove and the securing element, so that the ring at least partially covers the securing element, so as to prevent the securing element from disengaging from the insertion portion. Under the self-gravity of the medical device, the insertion portion is suspended on the securing element, which bears at least part of the weight of the medical device.

When the ring covers the securing element, the securing element is not easily disengaged under the cover of the ring. Therefore, the securing element is not easily disengaged from the insertion portion, and the medical device is not easily dropped, improving the reliability and safety of the medical device.

Medical devices may need to rotate during use, therefore, a fit between a securing element and an insertion portion allows the medical device to be connected with the support arm and rotated around an axis of the insertion portion, for example, for at least 360 °.

In a preferable embodiment, an outer circumference of the insertion portion is provided with a convex ring or a ring groove for limiting position; when the securing element abuts against a bottom of the convex ring or is inserted into the ring groove, the insertion portion is suspended on the securing element through the convex ring or the ring groove.

In a preferable embodiment, the support arm further includes a connection element, wherein the ring is connected with the connector through the connection element and is capable of being fixed at the second position through the connection element.

In a preferable embodiment, the connector includes a housing and an inner pipe, the housing is sleeved around an outer circumference of the inner pipe, the clamping groove penetrates through a side wall of the housing and a side wall of the inner pipe; wherein the side wall of the housing is provided with an avoidance groove, the ring is provided with a connection hole, one end of the connection element penetrates through the connection hole and the avoidance groove; wherein when said end of the connection element is connected with the inner pipe, the connection element fixes the ring at the second position, when said end of the connection element is separated from the inner pipe, the ring is capable of driving the connection element to move along the avoidance groove.

In a preferable embodiment, the connection element includes a first connection segment which is in a clearance fit with the connection hole, and a second connection segment which is connected with the inner pipe, wherein the second connection segment is capable of being in threaded engagement with the connection hole, so as to penetrate through the connection hole; wherein a width of the avoidance groove is greater than a diameter of the second connection segment, and when the second connection segment is separated from the inner pipe, the second connection segment is accommodated inside the avoidance groove.

In a preferable embodiment, the connection element includes a first connection segment which is in a clearance fit with the connection hole, and a second connection segment which is connected with the inner pipe, wherein the second connection segment is capable of being in threaded engagement with the connection hole, so as to penetrate through the connection hole; wherein at least at a position which is adjacent to the first connection segment, a diameter of the second connection segment is greater than that of the first connection segment; a length of the first connection segment is greater than a depth of a corresponding portion of the connection hole; and a length of the second connection segment is less than a distance between an inner wall of the ring and an outer wall of the inner pipe, but greater than a distance between the inner wall of the ring and an outer wall of the housing.

In a preferable embodiment, the connection element further includes an end cap, wherein the first connection segment is connected with the second connection segment and the end cap, wherein a sum of a length of the end cap and a length of the first connection segment is greater than a wall thickness of the ring.

In a preferable embodiment, the first connection segment is a non-threaded segment, the side wall of the inner pipe is provided with a threaded hole, the second connection segment is a threaded segment, which is configured to achieve threaded engagement with the threaded hole.

In a preferable embodiment, an aperture of the connection hole is greater than an outer diameter of the first connection segment; wherein a wall of the connection hole is provided with internal threads for threaded engagement with the second connection segment, when the second connection segment penetrates through the connection hole.

In a preferable embodiment, a distance between an outer wall of the inner pipe and an outer wall of the ring is less than a length of the connection element; wherein when the connection element is separated from the inner pipe, the connection element protrudes from the outer wall of the ring; when the connection element fixes the ring at the second position, the connection element does not extend beyond the outer wall of the ring.

In a preferable embodiment, the avoidance groove is an arc-shaped groove, which extends along a circumferential direction of the housing; the avoidance groove is a curved groove, which extends along a circumferential direction and an axial direction of the housing; or the avoidance groove includes multiple groove segments, which extend along a circumferential direction and an axial direction of the housing, and are connected with one another;
when the connection element is separated from the inner pipe, the connection element penetrates through and is arranged inside the avoidance groove, a fit between the avoidance groove and the connection element defines a movement trajectory of the ring.

In a preferable embodiment, the connector includes a first connector segment and a second connector segment, wherein an outer diameter of the second connector segment is less than an outer diameter of the first connector segment, a blocking shoulder is formed at a connection point between the first connector segment and the second connector segment, the ring surrounds an outer circumference of the second connector segment, an inner diameter of the ring is less than the outer diameter of the first connector segment, the clamping groove is arranged at the second connector segment; wherein when the ring moves along its axial direction to a position where the ring is in contact with the blocking shoulder and/or to a position where the ring is in contact with the medical device, the ring at least partially covers the securing element.

In a preferable embodiment, the support arm further includes a protective sleeve, which is sleeved around the outer circumference of the connector and is capable of moving along an axial direction of the ring; wherein the protective sleeve has an installation position and an avoidance position; at the installation position, the protective sleeve is sleeved around an outer circumference of the ring and covers the insertion groove; at the avoidance position, the protective sleeve exposes the insertion groove;
when the connection element is separated from the inner pipe, a portion of the connection element protrudes from an outer wall of the ring, so as to limit an axial movement of the protective sleeve.

In a preferable embodiment, the protective sleeve is capable of rotating relative to the ring and the connector.

In a preferable embodiment, a deformable snap-fit element is arranged on a circumferential outer surface of the connector, a first positioning groove is arranged on an inner wall of the protective sleeve; when the protective sleeve is located at the installation position, a portion of the deformable snap-fit element is snapped into the first positioning groove, so as to limit the protective sleeve at the installation position at least along an axial direction of the protective sleeve.

In a preferable embodiment, a second positioning groove is arranged on the inner wall of the protective sleeve; when the protective sleeve is located at the avoidance position, a portion of the deformable snap-fit element is snapped into the second positioning groove, so as to limit the protective sleeve at the avoidance position along the axial direction of the protective sleeve.

In a preferable embodiment, the deformable snap-fit element includes a deformable sheet and a convex portion which is arranged on an outer surface of the deformable sheet, wherein an end of the deformable sheet, which end is adjacent to the ring, is a free end; another end of the deformable sheet, which end is away from the ring, is a fixed end.

In a preferable embodiment, the connector includes a first connector segment and a second connector segment, wherein an outer diameter of the second connector segment is less than an outer diameter of the first connector segment, a blocking shoulder is formed at a connection point between the first connector segment and the second connector segment, the ring surrounds an outer circumference of the second connector segment, an inner diameter of the ring is less than the outer diameter of the first connector segment, the clamping groove is arranged at the second connector segment; the medical device is provided with a top surface which faces the blocking shoulder, wherein the top surface is in contact with the ring, so as to block the ring from continuing to move towards the medical device along an axial direction of the ring; a distance between the clamping groove and the blocking shoulder, as well as a distance between the clamping groove and the top surface, are both less than a length of the ring along said axial direction.

In a preferable embodiment, the length of the ring along said axial direction does not exceed a length of the second connector segment.

In a preferable embodiment, an outer diameter of the ring is not greater than the outer diameter of the first connector segment.

In a preferable embodiment, the support arm further includes a protective sleeve, which has an installation position; wherein at the installation position, the protective sleeve is sleeved around an outer circumference of the ring and covers the insertion groove.

In a preferable embodiment, a length of the protective sleeve along its axial direction is greater than a length of the ring along its axial direction; wherein when the protective sleeve is at the installation position, opposite ends of the ring along its axial direction do not extend beyond opposite ends of the protective sleeve along its axial direction.

In a preferable embodiment, a deformable snap-fit element is arranged on a circumferential outer surface of the connector, a first positioning groove is arranged on an inner wall of the protective sleeve; when the protective sleeve is located at the installation position, a portion of the deformable snap-fit element is snapped into the first positioning groove, so as to limit the protective sleeve at the installation position at least along an axial direction of the protective sleeve.

In a preferable embodiment, the protective sleeve is capable of moving upward along an axial direction of the ring to an avoidance position, at which position the insertion groove is exposed.

In a preferable embodiment, a deformable snap-fit element is arranged on an outer surface of the connector, a second positioning groove is arranged on an inner wall of the protective sleeve; when the protective sleeve is located at the avoidance position, a portion of the deformable snap-fit element is snapped into the second positioning groove, so as to limit the protective sleeve at the avoidance position at least along an axial direction of the protective sleeve.

In a preferable embodiment, the deformable snap-fit element includes a deformable sheet and a convex portion which is arranged on an outer surface of the deformable sheet, wherein an end of the deformable sheet, which end is adjacent to the ring, is a free end; another end of the deformable sheet, which end is away from the ring, is a fixed end.

In a preferable embodiment, the connector includes a first connector segment and a second connector segment, wherein the second connector segment is connected with a lower end of the first connector segment, an outer diameter of the second connector segment is less than an outer diameter of the first connector segment; the ring surrounds an outer circumference of the second connector segment, an inner diameter of the ring is less than the outer diameter of the first connector segment; an inner diameter of the protective sleeve is greater than the outer diameter of the first connector segment and greater than an outer diameter of the ring.

In a preferable embodiment, a length of the ring along its axial direction does not exceed a length of the second connector segment along its axial direction.

In a preferable embodiment, a length of the protective sleeve along its axial direction is greater than a length of the ring along its axial direction.

In a preferable embodiment, a blocking shoulder is formed at a connection point between the first connector segment and the second connector segment, the medical device is provided with a top surface which faces the blocking shoulder, wherein the top surface is configured to block and contact with the protective sleeve along its axial direction; a distance between the clamping groove and the blocking shoulder, as well as a distance between the clamping groove and the top surface, are both less than a length of the ring along said axial direction.

An embodiment of this disclosure provides a support arm, including:
an arm body, which is provided with a connector, wherein the connector is provided inside with an insertion cavity for inserting an insertion portion of a medical device; wherein a side wall of the connector is provided with a clamping groove;
a securing element;
a ring that is capable of preventing the securing element from disengaging from the insertion portion, wherein the ring is provided with an insertion groove, and movably sleeved around an outer circumference of the connector, so as to at least move between a first position and a second position;
when the ring is at the first position, the insertion groove is connected with the clamping groove, so as to enable the securing element to be inserted into the clamping groove through the insertion groove to engage with the insertion portion of the medical device; when the ring is at the second position, the ring at least partially covers the securing element, so as to prevent the securing element from disengaging from the insertion portion.

When assembling the support arm of this embodiment of this disclosure, the ring is sleeved around an outer circumference of the connector, the insertion portion of the medical device is inserted into the insertion cavity, the ring is moved to the first position, so as to align the insertion groove with the clamping groove, and then the securing element is inserted into the clamping groove through the insertion groove, and further inserted into the insertion portion. That is to say, a portion of the securing element is located inside the insertion portion and another portion of the securing element is located inside the clamping groove. Then, the ring is moved, so as to least partially misaligned the insertion groove and the securing element, so that the ring at least partially covers the securing element, so as to prevent the securing element from disengaging from the insertion portion. Under the self-gravity of the medical device, the insertion portion is suspended on the securing element, which bears at least part of the weight of the medical device.

When the ring covers the securing element, the securing element is not easily disengaged under the cover of the ring. Therefore, the securing element is not easily disengaged from the insertion portion, and the medical device is not easily dropped, improving the reliability and safety of the medical device.

Medical devices may need to rotate during use, therefore, a fit between a securing element and an insertion portion allows the medical device to be connected with the support arm and rotated about an axis of the insertion portion, for example, for at least 360 °.

In a preferable embodiment, the support arm further includes a connection element, wherein the ring is connected with the connector through the connection element and is capable of being fixed at the second position through the connection element.

In a preferable embodiment, the connector includes a housing and an inner pipe, the housing is sleeved around an outer circumference of the inner pipe, the clamping groove penetrates through a side wall of the housing and a side wall of the inner pipe; wherein the side wall of the housing is provided with an avoidance groove, the ring is provided with a connection hole, one end of the connection element penetrates through the connection hole and the avoidance groove; wherein when said end of the connection element is connected with the inner pipe, the connection element fixes the ring at the second position, when said end of the connection element is separated from the inner pipe, the ring is capable of driving the connection element to move along the avoidance groove.

In a preferable embodiment, the connection element includes a first connection segment which is in a clearance fit with the connection hole, and a second connection segment which is connected with the inner pipe, wherein the second connection segment is capable of being in threaded engagement with the connection hole, so as to penetrate through the connection hole; wherein a width of the avoidance groove is greater than a diameter of the second connection segment, and when the second connection segment is separated from the inner pipe, the second connection segment is accommodated inside the avoidance groove.

In a preferable embodiment, the connection element includes a first connection segment which is in a clearance fit with the connection hole, and a second connection segment which is connected with the inner pipe, wherein the second connection segment is capable of being in threaded engagement with the connection hole, so as to penetrate through the connection hole; wherein at least at a position which is adjacent to the first connection segment, a diameter of the second connection segment is greater than that of the first connection segment; a length of the first connection segment is greater than a depth of a corresponding portion of the connection hole; and a length of the second connection segment is less than a distance between an inner wall of the ring and an outer wall of the inner pipe, but greater than a distance between the inner wall of the ring and an outer wall of the housing.

In a preferable embodiment, the connection element further includes an end cap, wherein the first connection segment is connected with the second connection segment and the end cap, wherein a sum of a length of the end cap and a length of the first connection segment is greater than a wall thickness of the ring.

In a preferable embodiment, the first connection segment is a non-threaded segment, the side wall of the inner pipe is provided with a threaded hole, the second connection segment is a threaded segment, which is configured to achieve threaded engagement with the threaded hole.

In a preferable embodiment, an aperture of the connection hole is greater than an outer diameter of the first connection segment; wherein a wall of the connection hole is provided with internal threads for threaded engagement with the second connection segment, when the second connection segment penetrates through the connection hole.

In a preferable embodiment, a distance between an outer wall of the inner pipe and an outer wall of the ring is less than a length of the connection element; wherein when the connection element is separated from the inner pipe, the connection element protrudes from the outer wall of the ring; when the connection element fixes the ring at the second position, the connection element does not extend beyond the outer wall of the ring.

An embodiment of this disclosure provides a surgical light, including:
a light head assembly, which includes a light holder, a connection arm, and multiple light sources which sources are arranged at the light holder; wherein the light sources are configured to project illumination lights to a surgical area, and the light holder is connected with the connection arm; and
a support arm described in any embodiment of this disclosure; wherein the connection arm is connected with the insertion portion, and an outer circumference of the insertion portion is provided with a convex ring or a ring groove for limiting position; wherein when the securing element abuts against a bottom of the convex ring or is inserted into the ring groove, the light head assembly is suspended on the securing element through the insertion portion, so as to enable the light head assembly to be rotatable relative to the connector.

### BRIEF DESCRIPTION OF THE DRAWINGS

FIG. 1 is a diagram of a support arm according to an embodiment of this disclosure, in which a protective sleeve is at an avoidance position.
FIG. 2 is an exploded diagram of a connector and a protective sleeve according to an embodiment of this disclosure.
FIG. 3 is a diagram of a support arm and a connection arm of a medical device in an embodiment of this disclosure, wherein a protective sleeve is at an avoidance position and a securing element has not yet been inserted into a clamping groove.
FIG. 4 is an exploded diagram of a partial structure of a structure shown in FIG. 3 from another perspective.

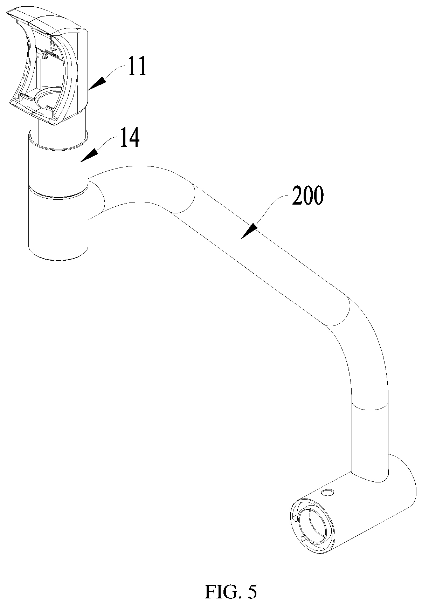

FIG. 5 is a diagram of another state of a structure shown in FIG. 3, wherein a protective sleeve is at an installation position.
FIG. 6 is a diagram from another perspective of a structure shown in FIG. 5, with a portion of a connection arm omitted, wherein a protective sleeve is at an installation position.
FIG. 7 is a diagram of the structure shown in FIG. 6 from another perspective, wherein a protective sleeve is at an avoidance position.
FIG. 8 is a diagram of a structure shown in FIG. 7 with a ring omitted.

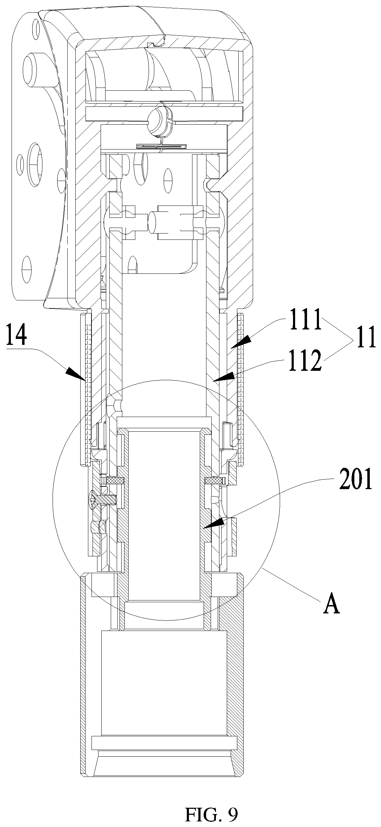

FIG. 9 is a sectional view of a structure shown in FIG. 8.
FIG. 10 is a partially enlarged diagram of point A in FIG. 9.
FIG. 11 is a sectional view of a structure shown in FIG. 8.
FIG. 12 is a diagram of a fit between a ring and a connection element according to an embodiment of this disclosure.

Description of Reference numbers.
100-Support arm;
11-Connector; 11A-First connector segment; 111A-Deformable snap-fit element; 1111 Deformable sheet; 1112-convex portion; 11B-Second connector segment; 11D-Blocking shoulder; 111-Housing; 111a-Avoidance groove; 112-Inner pipe; 112a-Threaded hole; 11a-Insertion cavity; 11b-Opening; 11c-Clamping groove;
12-Ring; 12a-Connection hole; 12b-First end surface along axial direction; 12c- Second end surface along axial direction; 12d-Insertion groove;
13-Connection element; 131-End cap; 132-First connection segment; 133-Second connection segment;
14-Protective sleeve; 14a-First positioning groove;
15-Securing element;
200-Connection arm; 201-Insertion portion; 200a-Top surface; 200b-Convex ring.

### DESCRIPTION OF THE PREFERRED EMBODIMENTS

In order to further clarify the purpose, technical solution, and advantages of this disclosure, the following provides a detailed explanation of this disclosure in conjunction with the accompanying drawings and embodiments. It should be understood that the specific embodiments described herein are only used to explain this disclosure and are not intended to limit this disclosure.

The various specific technical features described in the specific embodiments can be combined in any suitable way without contradiction, such as forming different embodiments and technical solutions through the combination of different specific technical features. In order to avoid unnecessary repetition, various possible combinations of specific technical features in this disclosure are not separately described.

In the following description, the terms "first\second\..." only distinguish different objects and do not imply similarities or connections between them. It should be understood that orientation descriptions "above", "below", "outside", and "inside" are all directions in normal use, and "left" and "right" directions represent the left and right directions indicated in the corresponding schematic diagram, which can be the left and right directions in normal use or not.

It should be noted that the terms "including", "containing", or any other variation thereof are intended to encompass non-exclusive inclusion, such that a process, method, article, or device that includes a series of elements not only includes those elements, but also includes other elements that are not explicitly listed or are inherent to such a process, method, article, or device. Without further limitations, the element defined by the statement 'including a...' does not exclude the existence of other identical elements in the process, method, article, or device that includes that element. "Multiple" means greater than or equal to two.

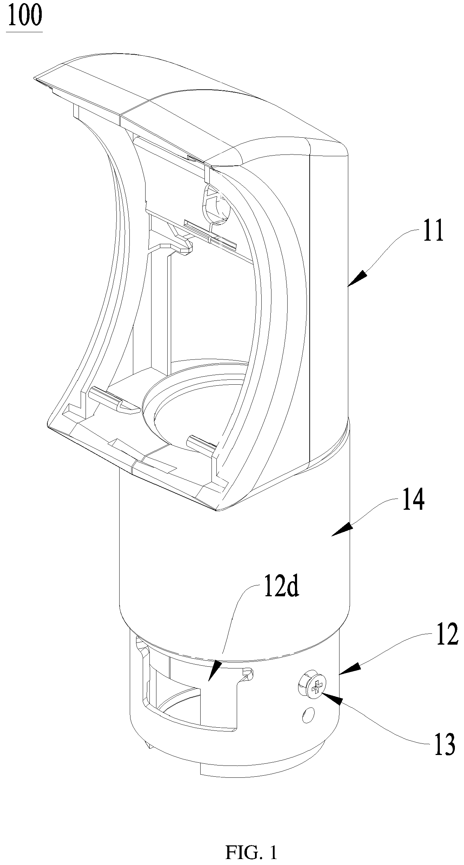

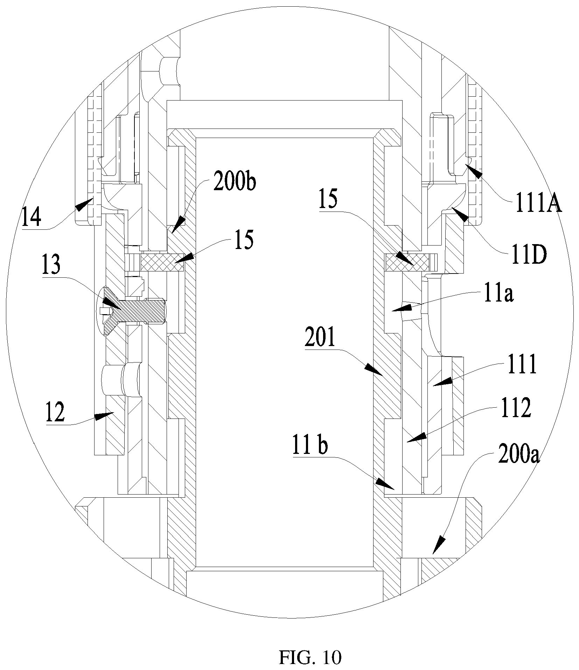

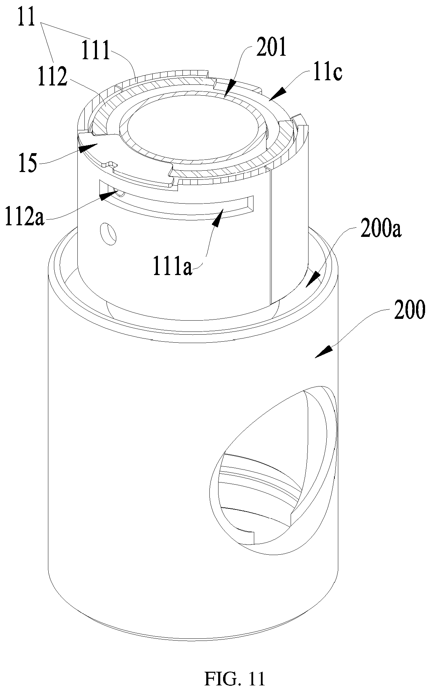

An embodiment of this disclosure provides a support arm 100, as shown in FIG. 1, including an arm body, a securing element 15 (referring FIGS. 10 and 11), a ring 12, and an insertion portion 201 for connecting a medical device, wherein the ring 12 is capable of preventing the securing element 15 from disengaging from the insertion portion 201.

The arm body is provided with a connector 11, which is configured to connect a medical device. The specific type of the medical device is not limited. For example, it could be a light head assembly of a surgical light.

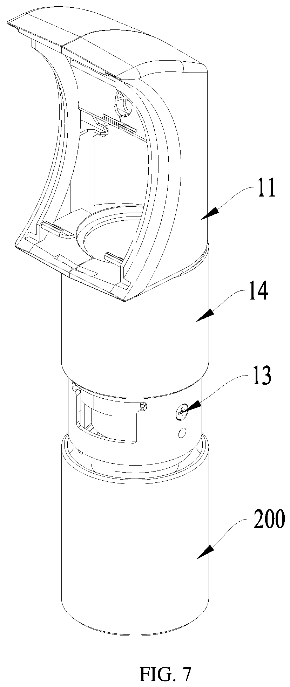

Please refer to FIG. 10. The connector 11 is provided inside with an insertion cavity 11a for inserting the insertion portion 201. Specifically, a lower end of the insertion cavity 11a is provided with an opening 11b, and the insertion portion 201 is inserted into the insertion cavity 11a through the opening 11b. Please refer to FIGS. 7 and 11, a side wall of connector 11 is provided with a clamping groove 11c.

The ring 12 is movably sleeved around an outer circumference of the connector 11, so as to at least move between a first position and a second position.

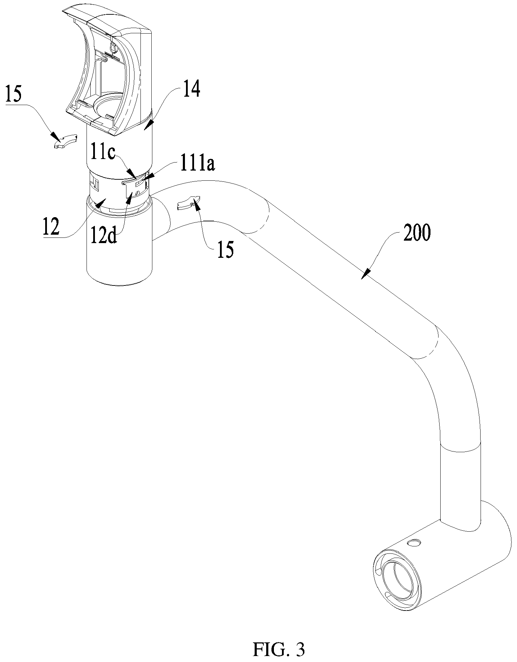

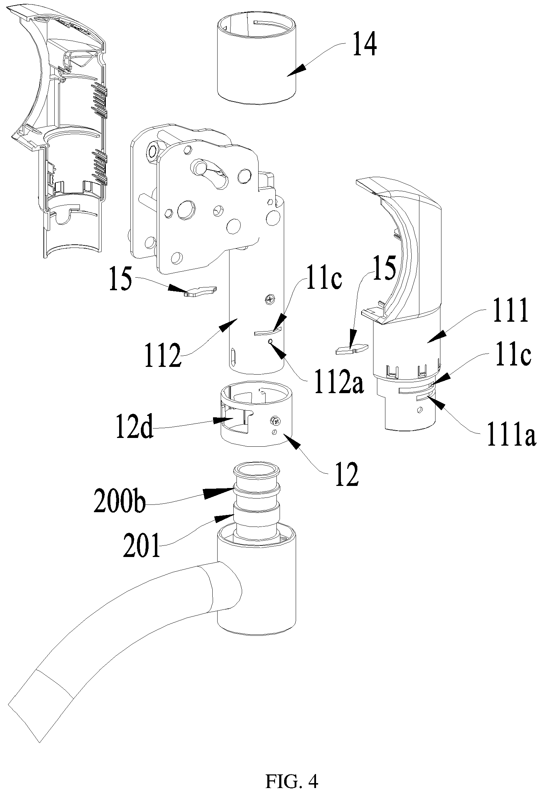

Please refer to FIGS. 3 and 4, the ring 12 has an insertion groove 12d. Specifically, the insertion groove 12d penetrates through a side wall of the ring 12 along a radial direction of the ring 12.

When the ring 12 is at the first position, the insertion groove 12d is connected with the clamping groove 11c, so as to enable the securing element 15 to be inserted into the clamping groove 11c through the insertion groove 12d to engage with the insertion portion 201, so as to further enable the insertion portion 201 to be suspended on the securing element 15 and to be capable of rotating relative to the connector 11.

When the ring 12 is at the second position, the ring 12 at least partially covers the securing element 15, so as to prevent the securing element 15 from disengaging from the insertion portion 201.

When assembling the support arm 100 of this embodiment, the ring 12 is sleeved around an outer circumference of the connector 11, the insertion portion 201 is inserted into the insertion cavity 11a, the ring 12 is moved to the first position, so as to align the insertion groove 12d with the clamping groove 11c, and then the securing element 15 is inserted into the clamping groove 11c through the insertion groove 12d, and further inserted into the insertion portion 201. That is to say, a portion of the securing element 15 is located inside the insertion portion 201, and another portion of the securing element 15 is located inside the clamping groove 11c. Then, the ring 12 is moved, which causes the insertion groove 12d and the securing element 15 to be at least partially misaligned, so as to enable the ring 12 to at least partially cover the securing element 15, and to prevent the securing element 15 from disengaging from the insertion portion 201. Under the self-gravity of the medical device, the medical device is suspended on the securing element 15 through the insertion portion 201, and the securing element 15 bears at least part of the weight of the medical device.

It should be noted that the aforementioned movement includes translation and/or rotation.

When the ring 12 covers the securing element 15, the securing element 15 is not easily disengaged under the cover of the ring 12. Therefore, the securing element 15 is not easily disengaged from the insertion portion 201, and the medical device is not easily to fall down, improving the reliability and safety of the medical device.

Medical device may need to rotate during use, therefore, a fit between the securing element 15 and the insertion portion 201 allows the medical device to be connected with the support arm 100 and rotated about an axis of the insertion portion 201, for example, for at least 360 °.

A shape of the securing element 15 is adaptive with that of the clamping groove 11c, and both of the securing element 15 and the clamping groove 11c have a similar shape and size.

In some embodiments, the clamping groove 11c extends approximately along a circumference direction of the connector 11, that is, approximately forming a fan-ring shape, and the securing element 15 also approximately forms a fan-ring shape.

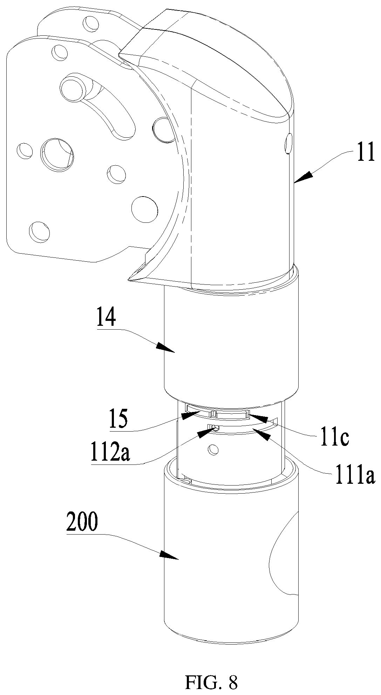

For example, referring to FIGS. 1 and 8, the support arm 100 includes a connection element 13, and the ring 12 is connected with the connector 11 through the connection element 13 and is capable of being fixed at the second position by the connection element 13.

In this embodiment, when the ring 12 is at the second position, it is fixed to the connector 11 through the connection element 13. At this time, there will be no relative movement between the ring 12 and the connector 11, so that the ring 12 covers the securing element 15 and reduces the probability of disengagement for the securing element 15 through the clamping groove 11c. It should be noted that when the ring 12 is at the first position, the ring 12 can also be connected with the connection element 13, but the connection element 13 allows the ring 12 to rotate relative to the connector 11. Of course, at the first position, the connection element 13 can also be completely removed from the ring 12.

By way of example, please refer to FIGS. 4, 10, and 11. The connector 11 includes a housing 111 and an inner pipe 112. The housing 111 is sleeved around an outer circumference of the inner pipe 112, and the clamping groove 11c penetrates through side walls of the housing 111 and of the inner pipe 112. That is to say, the securing element 15 passes through the side walls of the housing 111 and of the inner pipe 112.

In some embodiments, materials of the inner pipe 112, the insertion portion 201, and the securing element 15 may all be metal. The insertion portion 201 is inserted into the inner pipe 112, wherein the inner pipe 112, the securing element 15, and the insertion portion 201 all serve as structural load-bearing elements. The metal material can enhance structural strength and reliability.

Material of the housing 111 can be plastic. The housing 111 serves as an exterior element of the support arm 100, covering the structure of the inner pipe 112 and facilitating the formation of the required process shape.

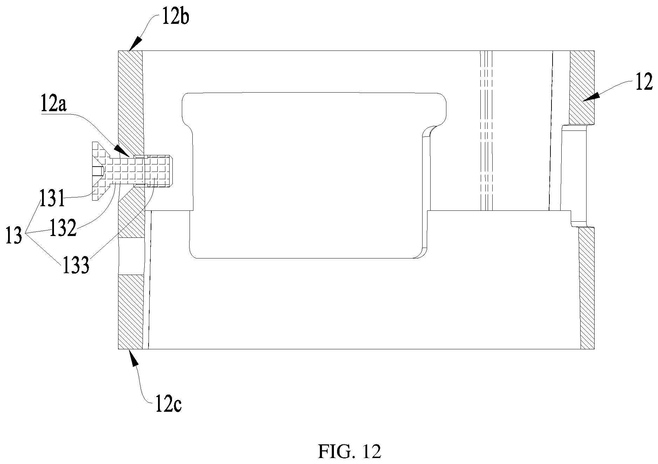

By way of example, please refer to FIGS. 4 and 10. The side wall of the housing 111 is provided with an avoidance groove 111a. Please refer to FIG. 12. The ring 12 is provided with a connection hole 12a, and one end of the connection element 13 penetrates through the connection hole 12a and the avoidance groove 111a. When one end of the connection element 13 is connected with the inner pipe 112, the connection element 13 fixes the ring 12 at the second position. When one end of the connection element 13 is separated from the inner pipe 112, the ring 12 can drive the connection element 13 to move along the avoidance groove 111a. The inner pipe 112 serves as a structural load-bearing element, and the connection element 13 is fixed to the inner pipe 112, ensuring a reliable structure. It should be noted that when the connection element 13 is fixed to the inner pipe 112, the ring 12 at least partially covers the securing element 15.

The specific type of the connection element 13 is not limited, for example, it can be a screw.

For example, referring to FIG. 12, the connection element 13 includes a first connection segment 132 and a second connection segment 133. The first connection segment 132 is used for a clearance fit with the connection hole 12a, the second connection segment 133 is used for connection with the inner pipe 112.

For example, the second connection segment 133 can be in threaded engagement with the connection hole 12a, so as to penetrate through the connection hole 12a, that is to say, the second connection segment 133 is a threaded segment. Preferably, a width of the avoidance groove 111a along an axial direction of the ring 12 is greater than a diameter of the second connection segment 133. When the second connection segment 133 of the connection element 13 is separated from the inner pipe 112, the second connection segment 133 is accommodated inside the avoidance groove 111a. At this time, the second connection segment 133 is supported by the avoidance groove 111a. Specifically, when the second connection segment 133 of the connection element 13 is separated from the inner pipe 112, the ring 12 falls with the connection element 13 under the gravity of the ring 12, and then is supported by the avoidance groove 111a. In addition, the ring 12 exerts a downward force on the connection element 13. Therefore, a height of an end of the second connection segment 133, which end is adjacent to the ring 12, is lower than a height of an end of the second connection segment 133, which end is away from the ring 12, which is equivalent to that, the second connection segment 133 swings for a certain angle inside the avoidance groove 111a. This angle makes it difficult for a step structure to be aligned with the connection hole 12a. Without alignment, even if the connection element 13 is screwed, the second connection segment 133 is difficult to enter the connection hole 12a. In other words, it is difficult for the connection element 13 to be disengaged from the connection hole 12a, thus improving the reliability of disengagement prevention of the connection element 13. However, this disclosure is not limited to this, and other situations may be possible with reasonable settings for the size of the connection hole 12a, the width of the avoidance groove 111a, and the weight of the second connection segment 133. For example, the second connection segment 133 can be arranged to have a larger weight. When the second connection segment 133 of the connection element 13 is separated from the inner pipe 112; a height of an end of the second connection segment 133, which end is adjacent to the ring 12, is higher than a height of another end of the second connection segment 133, which end is away from the ring 12, or a height of an end of the second connection segment 133, which end is adjacent to the ring 12, is equal to a height of another end of the second connection segment 133, which end is away from the ring 12. That is, the connection element 13 falls down.

In some embodiments, the connection element 13 further includes an end cap 131, and the first connection segment 132 is connected with the second connection segment 133 and the end cap 131. That is to say, the end cap 131 and the second connection segment 133 are respectively connected with opposite ends of the first connection segment 132. Of course, in other embodiments, the connection element 13 may not have an end cap 131.

It should be noted that a minimum aperture of the connection hole 12a is greater than an outer diameter of the first connection segment 132, and the minimum aperture of the connection hole 12a is adapted to an outer diameter of the second connection segment 133, such that the second connection segment 133 and the first connection segment 132 can penetrate through the connection hole 12a in sequence.

At least at a position which is adjacent to the first connection segment 132, a diameter of the second connection segment 133 is greater than that of the first connection segment 132, so as to form a stepped structure at a junction between the first connection segment 132 and the second connection segment 133.

For example, a length of the first connection segment 132 is greater than a depth of a corresponding portion of the connection hole 12a; and a length of the second connection segment 133 is less than a distance between an outer wall of the inner pipe 112 and an inner wall of the ring 12, but greater than a distance between the inner wall of the ring 12 and an outer wall of the housing 111.

Wherein, the depth of the corresponding portion of the connection hole 12a refers to a depth of a portion of the connection hole 12a, which portion engages with the first connection segment 132 after the connection element 13 is fixed to the inner pipe 112. For example, in the embodiment where the connection element 13 has an end cap 131, the connection hole 12a includes a first portion and a second portion, wherein the first portion is a conical hole, i.e., funnel-shaped, and an inner diameter of an inner end of the first portion along a radial direction of the ring 12 is less than an inner diameter of an outer end of the first portion, while the second portion is a straight hole with an equal diameter. When the connection element 13 is fixed to the inner pipe 112, the end cap 131 is accommodated inside the first portion, and the first connection segment 132 is accommodated inside the second portion. At this time, the depth of the corresponding portion of the connection hole 12a refers to a depth of the second portion. For example, in the embodiment where the connection element 13 does not have an end cap 131, when the connection element 13 is fixed to the inner pipe 112, a length of the connection element 13 inside the connection hole 12a is a depth of the corresponding portion of the connection hole.

In this embodiment, when the connection element 13 is fixed to the inner pipe 112, at least a portion of the second connection segment 133 is inserted into the inner pipe 112, the first connection segment 132 penetrates through and is arranged into the connection hole 12a. When the connection element 13 is separated from the inner pipe 112, the second connection segment 133 moves into the avoidance groove 111a. At this time, the second connection segment 133 does not interfere with the inner pipe 112. When the ring 12 rotates, the second connection segment 133 slides inside the avoidance groove 111a, so as to enable the ring 12 to drive the connection element 13 to rotate relative to the connector 11.

Due to that the stepped structure is formed at the junction between the second connection segment 133 and the first connection segment 132, the stepped structure and a wall of the connection hole 12a have a blocking effect to a certain extent, which reduces the probability of disengagement for the second connection segment 133 through the connection hole 12a, thereby making it difficult for the connection element 13 to be disengaged from the ring 12.

Preferably, a sum of a length of the first connection segment 132 and a length of the end cap 131 is greater than a wall thickness of the ring 12.

For example, the first connection segment 132 is a non-threaded segment, such as a smooth rod-segment. Please refer to FIGS. 4 and 11. The side wall of the inner pipe 112 is provided with a threaded hole 12a, and the second connection segment 133 is a threaded segment which is configured to achieve threaded engagement with the threaded hole 12a. In this way, by screwing the connection element 13, the connection element 13 can be fixed to the inner pipe 112. Specifically, a threaded hole 112a is located within the range of the avoidance groove 111a. When the second connection segment 133 is moved to be aligned with the threaded hole 112a, the second connection segment 133 can be screwed into the threaded hole 112a.

For example, a wall of the connection hole 12a is provided with internal threads for threaded engagement with the second connection segment 133, when the second connection segment 133 penetrates through the connection hole 12a. Specifically, when the connection element 13 penetrates through the connection hole 12a in a radial direction from outside to inside, due to the internal threaded engagement between the second connection segment 133 and the connection hole 12a, it is necessary to continuously screw the connection element 13, so as to enable the second connection segment 133 to completely penetrate through the connection hole 12a. Afterwards, the first connection segment 132 penetrates through and is arranged into the connection hole 12a. When the second connection segment 133 is withdrawn from the threaded hole 112a of the inner pipe 112, the second connection segment 133 enters the avoidance groove 111a, then the ring 12 and the connection element 13 are supported by the avoidance groove 111a and the connection hole 12a. Due to that the threads of the second connection segment 133 and the internal threads of the connection hole 12a should be aligned and continuously screwed outward, so as to enable the second connection segment 133 to enter into the connection hole 12a, the weight of the ring 12 itself acts on the connection element 13 to generate a bending moment on the connection element 13, making it difficult for the threads of the second connection segment 133 and the internal threads of the connection hole 12a to be aligned. Without alignment, even if the connection element 13 is screwed, the second connection segment 133 still cannot enter the connection hole 12a, which means it is difficult for the connection element 13 to be disengaged from the connection hole 12a, thus improving the reliability of disengagement prevention of the connection element 13.

It should be noted that the end cap 131 is used to abut against the ring 12, in order to exert a force on the ring 12, which force is towards the connector 11.

For example, a distance between an outer wall of the inner pipe 112 and an outer wall of the ring 12 is less than a length of the connection element 13. When the connection element 13 is separated from the inner pipe 112, the connection element 13 protrudes from the outer wall of the ring 12. For example, in an embodiment with an end cap 131, the end cap 131 protrudes from the outer wall of the ring 12. When the connection element 13 fixes the ring 12 at the second position, the connection element 13 does not extend beyond the outer wall of the ring 12. In this way, the connection element 13 is prevented from scratching or interfering with other structures.

It should be noted that when the connection element 13 is separated from the inner pipe 112, and the connection element 13 penetrates through and is arranged into the avoidance groove 111a, a fit between the avoidance groove 111a and the connection element 13 defines a movement trajectory of the ring 12. Specifically, when the ring 12 rotates, the ring 12 drives the connection element 13 to move together, and the connection element 13 moves inside the avoidance groove 111a. Therefore, the shape of the avoidance groove 111a determines the movement trajectory of the connection element 13, which in turn determines the movement trajectory of the ring 12.

In some embodiments, the avoidance groove 111a is an arc-shaped groove which extends along a circumferential direction of the housing 111. When the ring 12 rotates, the connection element 13 moves inside the avoidance groove 111a along the circumferential direction without any axial displacement. Therefore, the ring 12 only rotates in the circumferential direction without any axial displacement.

In other embodiments, the avoidance groove 111a is a curved groove which extends along both circumferential and axial directions of the housing 111. That is to say, the avoidance groove 111a can be understood as a spirally extended groove along a surface of the housing 111. When the ring 12 rotates, the connection element 13 moves inside the avoidance groove 111a along both the circumferential direction and the axial direction. Therefore, the ring 12 also rotates in the circumferential direction and meanwhile moves along the axial direction.

In another embodiment, the avoidance groove 111a includes multiple groove segments, which extend along circumferential and axial directions of the housing 111 and are connected with one another, such as a first groove segment which extends along the axial direction of the housing 111 and a second groove segment which extends along the circumferential direction of the housing 111. When the ring 12 moves, the connection element 13 first moves inside the avoidance groove 111a along the axial direction, and then along the circumferential direction.

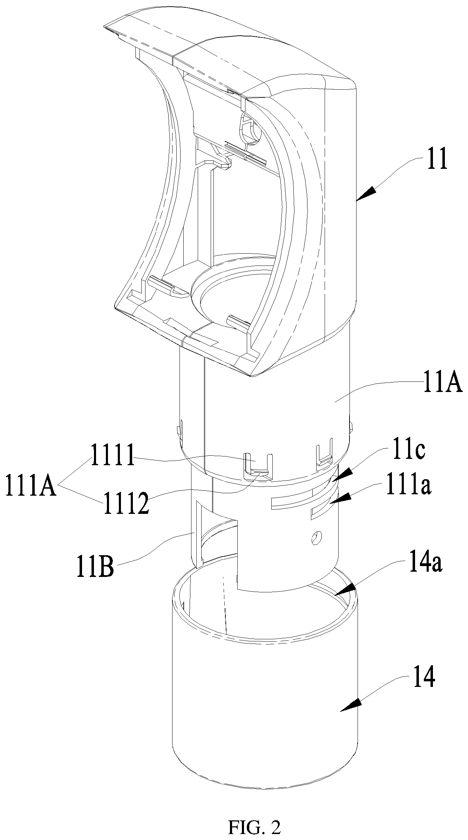

For example, referring to FIG. 2, the connector 11 includes a first connector segment 11A and a second connector segment 11B; wherein an outer diameter of the second connector segment 11B is less than that of the first connector segment 11A. The second connector segment 11B is located at a lower end of the first connector segment 11A. Specifically, the opening 11b is located at the lower end of the second connector segment 11B.

A blocking shoulder 11D is formed at a connection point between the first connector segment 11A and the second connector segment 11B (refer to FIG. 10). The ring 12 surrounds an outer circumference of the second connector segment 11B, and the inner diameter of the ring 12 is less than the outer diameter of the first connector segment 11A, which means that the ring 12 never moves to the outer circumference of the first connector segment 11A. The clamping groove 11c is arranged at the second connector segment 11B.

It should be noted that the first connector segment 11A and the second connector segment 11B are distinguished based on the shape of the connector 11, and both the first connector segment 11A and the second connector segment 11B may include the housing 111 and the inner pipe 112. That is to say, a portion of the inner pipe 112 is located inside the first connector segment 11A, another portion of the inner pipe 112 is located inside the second connector segment 11B, and a portion of the housing 111 is located inside the first connector segment 11A, another portion of the housing 111 is located inside the second connector segment 11B. The connection element 13 is used to connect with the inner pipe 112 which corresponds to the second connector segment 11B.

Wherein, an outer diameter of a portion of the housing 111, which portion is located at the first connector segment 11A, is greater than an outer diameter of another portion of the housing 111, which portion is located at the second connector segment 11B, so as to form the aforementioned blocking shoulder 11D outside the housing 111. It should be noted that, inner and outer diameters of portions of the inner pipe 112, which portions are respectively located at the first connector segment 11A and the second connector segment 11B, are not limited, as long as they can facilitate the insertion of the insertion portion 201.

The insertion portion 201 is inserted into the second connector segment 11B through the opening 11b at a bottom end of the second connector segment 11B, and fixed to the inner pipe 112 which corresponds to the second connector segment 11B. It should be noted that, a top end of the insertion portion 201 can also extend upwards into the inner pipe 112 which corresponds to the first connector segment 11A.

For example, when the ring 12 moves along its axial direction to a position where the ring 12 is in contact with the blocking shoulder 11D and/or to a position where the ring 12 is in contact with the medical device, the ring 12 at least partially covers the securing element 15.

For example, when the ring 12 moves along its axis to a position where the ring 12 is in contact with the blocking shoulder 11D, the ring 12 at least partially covers the securing element 15. Specifically, when the connection element 13 is separated from the second connector segment 11B, that is, the connection element 13 never penetrates through and is not arranged inside the avoidance groove 111a, for example, the connection element 13 may not have been connected with the ring 12, at this time, a movement of the ring 12 is not limited by the connection element 13, and the ring 12 can rotate relative to the second connector segment 11B, or move relative to the second connector segment 11B along the axial direction. That is to say, when the ring 12 moves along the axial direction to the position where the ring 12 is in contact with the blocking shoulder 11D, positions of the clamping groove 11c and the securing element 15 are misaligned by a certain distance along the axial direction. Therefore, no matter how the ring 12 rotates, the ring 12 always covers at least a portion of the securing element 15, and the securing element 15 cannot be aligned with the clamping groove 11c. Therefore, the securing element 15 cannot be disengaged.

It should be noted that, in the case where the connection element 13 is connected with the second connector segment 11B (for example, including two cases: the second connection segment 133 mentioned above can be fixed to the inner pipe 112, or the second connection segment 133 can be separated from the inner pipe 112 and fall into the avoidance groove 111a), the ring 12 may not be in contact with the blocking shoulder 11D.

For further example, when the connection element 13 is separated from the second connector segment 11B and the ring 12 moves along the axial direction to contact with the connection arm 200 of the medical device, the ring 12 at least partially covers the securing element 15. In the case when the connection element 13 is separated from the second connector segment 11B, that is, the connection element 13 never penetrates through and is not arranged inside the avoidance groove 111a, for example, the connection element 13 may not have been connected with the ring 12, at this time, a movement of the ring 12 is not limited by the connection element 13, and the ring 12 can rotate relative to the second connector segment 11B, or move relative to the second connector segment 11B along the axial direction. That is to say, at this position, positions of the clamping groove 11c and the securing element 15 are misaligned by a certain distance along the axial direction. Therefore, no matter how the ring 12 rotates, the ring 12 always covers at least a portion of the securing element 15, and the securing element 15 cannot be aligned with the clamping groove 11c. Therefore, the securing element 15 cannot be disengaged.

It should be noted that, in the case where the connection element 13 is connected with the second connector segment 11B (for example, including two cases: the second connection segment 133 mentioned above can be fixed to the inner pipe 112, or the second connection segment 133 can be separated from the inner pipe 112 and fall into the avoidance groove 111a), the ring 12 may not be in contact with the connection arm 200.

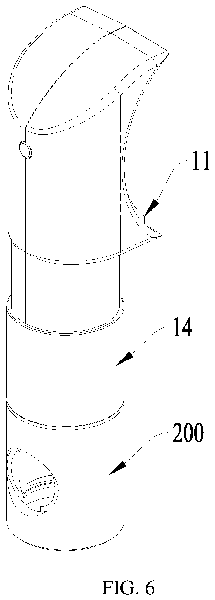

For example, the support arm 100 includes a protective sleeve 14, which is sleeved around the outer circumference of the connector 11 and capable of moving along the axial direction of the ring 12. The protective sleeve 14 has an installation position and an avoidance position. At the installation position, please refer to FIG. 6. The protective sleeve 14 shown in FIG. 6 is located at the installation position, and the protective sleeve 14 is sleeved around the outer circumference of the ring 12. At the avoidance position, please refer to FIGS. 7 and 8. The protective sleeve 14 shown in FIGS. 7 and 8 is located at the avoidance position, exposing the insertion groove 12d of the ring 12. That is to say, an inner diameter of the protective sleeve 14 is greater than an outer diameter of the ring 12, and the protective sleeve 14 can move to the outer circumference of the ring 12 along the axial direction.

When the protective sleeve 14 is at the avoidance position, the protective sleeve 14 can be moved to a position where the protective sleeve 14 does not interfere with the assembly of the ring 12. The protective sleeve 14 and the ring 12 can completely never overlap along the axial direction, or can partially overlap along the axial direction. At this time, a portion of the protective sleeve 14 is sleeved around an outer circumference of a portion of the ring 12.

For example, during the assembly process, the protective sleeve 14 can be first placed at the avoidance position, the ring 12 can be assembled at first, and then the protective sleeve 14 can be moved to the outer circumference of the ring 12.

In this embodiment, during the use of medical device, the protective sleeve 14 is located at the outer circumference of the ring 12. Due to that the protective sleeve 14 is sleeved around the outer circumference of the ring 12, even if the securing element 15 is aligned with the clamping groove 11c due to malfunction of the ring 12, the securing element 15 is still not disengaged from the clamping groove 11c due to the blocking of the protective sleeve 14. That is, the securing element 15 never comes out, such that the medical device never falls down. The protective sleeve 14 can further prevent the securing element 15 from disengagement, further improving the safety of the support arm 100.

In the embodiment with the connection element 13, the protective sleeve 14 is also used to block the connection element 13, which can prevent the connection element 13 from moving outward in a radial direction, thereby keeping the connection element 13 to be fixed to the inner pipe 112, which is conducive to fixing the ring 12 at the second position and reducing the probability of disengagement for the securing element 15.

For example, in the embodiment provided with the connection element 13, when the connection element 13 is separated from the inner pipe 112, a portion of the connection element 13 protrudes from the outer wall of the ring 12 to limit an axial movement of the protective sleeve 14. Specifically, during the assembly process, if the connection element 13 is not yet fixed to the inner pipe 112, or if the connection element 13 is not screwed in place, a portion of the connection element 13 protrudes from the outer wall of the ring 12. At this time, if the protective sleeve 14 is wanted to be moved to the outer circumference of the ring 12, the protective sleeve 14 will interfere with the connection element 13, and the protective sleeve 14 cannot be moved to the installation position. In this way, it can serve as a reminder to the operator that the connection element 13 must be installed in place before the protective sleeve 14 can be moved in place, playing an auxiliary role in ensuring that the connection element 13 is installed in place.

For example, the protective sleeve 14 is capable of rotating relative to the ring 12 and the connector 11. That is to say, whether during or after the assembly process, the protective sleeve 14 will never be fixed to the ring 12 or the connector 11, and the protective sleeve 14 can always rotate and move under an external force.

This solution does not require use of screws to fix the protective sleeve 14, thus achieving a screw free appearance of the protective sleeve 14 and enhancing its aesthetic appeal. In addition, it is also convenient to disinfect the protective sleeve 14.

For example, the material of protective sleeve 14 is plastic. In this way, the plastic material facilitates the protective sleeve 14 to achieve more process appearance.

It should be noted that, in some embodiments, the support arm 100 is provided with both a connection element 13 and a protective sleeve 14. In other embodiments, the support arm 100 is provided with a connection element 13, but not provided with a protective sleeve 14. In some other embodiments, the support arm 100 is provided with a protective sleeve 14, but not provided with a connection element 13.

In the embodiment where the connector 11 includes a first connector segment 11A and a second connector segment 11B, an inner diameter of the protective sleeve 14 is greater than an outer diameter of the first connector segment 11A. When the protective sleeve 14 is at the installation position, the protective sleeve 14 is sleeved around outer circumferences of the second connector segment 11B and the ring 12. When the protective sleeve 14 is at the avoidance position, the protective sleeve 14 moves to an outer circumference of the first connector segment 11A.

In some embodiments, an outer wall of the ring 12 is approximately flush with an outer wall of the first connector segment 11A.

For example, a deformable snap-fit element 111A is provided on a circumferential outer surface of the connector 11. In some specific embodiments, a deformable snap-fit element 111A is provided on the circumferential surface of one end of the first connector segment 11A, which end is adjacent to the second connector segment 11B.

An inner wall of protective sleeve 14 is provided with a first positioning groove. When the protective sleeve 14 is at the installation position, a portion of the deformable snap-fit element 111A is snapped into the first positioning groove, so as to limit the protective sleeve 14 at the installation position at least along the axial direction.

It should be noted that the shape of the first positioning groove 14a is not limited. In some embodiments, when the first positioning groove 14a and a portion of the deformable snap-fit element 111A, which portion is snapped into the first positioning groove 14a, have a basically same dimension along the circumferential direction; the protective sleeve 14 cannot rotate relative to the connector 11 at the installation position. When the first positioning groove 14a extends for a certain length along the circumferential direction of the connector 11, the protective sleeve 14 can rotate relative to the connector 11 for a certain angle at the installation position, which angle depends on a central angle which corresponds to the first positioning groove 14a. For example, in some embodiments, the first positioning groove 14a may be a ring groove, that is, the first positioning groove 14a surrounds a circumferential inner surface of the protective sleeve 14 by 360 degrees, and the protective sleeve 14 can rotate freely in the circumferential direction.

Due to a position fit between the deformable snap-fit element 111A and the first positioning groove 14a, if it is necessary to move the protective sleeve 14 along the axial direction, the external force needs to overcome the force exerted by the deformable snap-fit element 111A on the protective sleeve 14 before to move the protective sleeve 14. From this, it can be seen that the deformable snap-fit element 111A can improve the stability of the protective sleeve 14 at the installation position, making it less likely for the protective sleeve 14 to be disengaged from the installation position in case of accidental touch.

For example, the inner wall of the protective sleeve 14 is provided with a second positioning groove. When the protective sleeve 14 is at the avoidance position, a portion of the deformable snap-fit element 111A is snapped into the second positioning groove to limit the protective sleeve 14 at the avoidance position at least along the axial direction.

It should be noted that the shape of the second positioning groove is not limited. In some embodiments, when the second positioning groove and a portion of the deformable snap-fit element 111A, which portion is snapped into the second positioning groove, have a basically same dimension along the circumferential direction; the protective sleeve 14 cannot rotate relative to the connector 11 at the installation position. When the second positioning groove extends for a certain length along the circumferential direction of the connector 11, the protective sleeve 14 can rotate relative to the connector 11 for a certain angle at the avoidance position, which angle depends on a central angle which corresponds to the second positioning groove. For example, in some embodiments, the second positioning groove may be a ring groove, that is, the second positioning groove surrounds a circumferential inner surface of the protective sleeve 14 by 360 degrees, and the protective sleeve 14 can rotate freely in the circumferential direction.

Due to a position fit between the deformable snap-fit element 111A and the second positioning groove, if it is necessary to move the protective sleeve 14 along the axial direction, the external force needs to overcome the force exerted by the deformable snap-fit element 111A on the protective sleeve 14 before to move the protective sleeve 14. From this, it can be seen that the deformable snap-fit element 111A can improve the stability of the protective sleeve 14 at the avoidance position. For example, during the assembly process, after moving the protective sleeve 14 to the avoidance position and releasing it, the protective sleeve 14 will not fall off on its own. After assembling the ring 12, a force is exerted to move the protective sleeve 14 to the outer circumference of the ring 12.

When the protective sleeve 14 is provided with both a first positioning groove 14a and a second positioning groove, the first positioning groove 14a is located inside the protective sleeve 14 and adjacent to an upper end of the protective sleeve 14; the second positioning groove is located inside the protective sleeve 14 and adjacent to a lower end of the protective sleeve 14.

The specific structure of the deformable snap-fit element 111A is not limited.

For example, referring to FIG. 2, the deformable snap-fit element 111A includes a deformable sheet 1111 and a convex portion 1112 which is arranged on an outer surface of the deformable sheet 1111. An end of the deformable sheet 1111, which end is adjacent to the ring 12, is a free end, and another end of the deformable sheet 1111, which end is away from the ring 12, is a fixed end. The convex portion 1112 is arranged at an outer side of the free end of the convex portion 1111. In this embodiment, when the protective sleeve 14 is at the installation position, if it is desired to move the protective sleeve 14 from the installation position to the avoidance position, and an external force, which is along the axial direction and away from the ring 12, is exerted to the protective sleeve 14, due to that the free end of the deformable sheet 1111 is adjacent to the ring 12, the deformable sheet 1111 tends to wedge against the ring 12. Therefore, a larger force is required to move the protective sleeve 14 away from the installation position. When the protective sleeve 14 is at the avoidance position, if an external force, which is along the axial direction and toward the ring 12, is exerted to the protective sleeve 14, the deformable sheet 1111 has a certain guiding effect. Therefore, a relatively small external force is required to move the protective sleeve 14 from the avoidance position to the installation position.

That is to say, the structure of the deformable sheet 1111 ensures that the external force required to move the protective sleeve 14 from the installation position to the avoidance position is greater than the external force required to move from the avoidance position to the installation position.

The specific shape of the convex portion 1112 is not limited, for example, it can be a hemispherical convex portion or a hook-shape convex portion, and is not limited here.

Please refer to FIGS. 10 and 11. The connection arm 200 of the medical device has a top surface 200A which faces the blocking shoulder 11D. The top surface 200A of the medical device is in contact with the ring 12 to prevent it from continuing to move along the axial direction towards the medical device.

A distance between the clamping groove 11c and the blocking shoulder 11D, as well as a distance between the clamping groove 11c and the top surface 200A of the medical device, may be less than a length of the ring 12 along its axial direction.

The distance between the clamping groove 11c and the blocking shoulder 11D refers to an axial distance between the blocking shoulder 11D and a groove wall at a side of the clamping groove 11c, which side is adjacent to the blocking shoulder 11D. The distance between the clamping groove 11c and the top surface 200A of the medical device refers to an axial distance between the top surface 200A of the medical device and a groove wall at another side of the clamping groove 11c, which side is adjacent to the top surface 200A of the medical device.

For ease of description, please refer to FIG. 12. An end surface of the ring 12 along the axial direction, which surface faces the blocking shoulder 11D, is referred to as a first end surface 12b along the axial direction, another end surface of the ring 12 along the axial direction, which surface faces the top surface 200A of the medical device is referred to as a second end surface 12c along the axial direction. The first end surface 12b along the axial direction and the second end surface 12c along axial direction are opposite end surfaces of the ring 12 along the axial direction.

For example, when the position of the ring 12 has not been fixed to the second connector segment 11B, even if the ring 12 can move along the axial direction, when the ring 12 moves towards the blocking shoulder 11D, so as to enable the first end surface 12b of the ring 12 to contact with the blocking shoulder 11D, the ring 12 will not be disengaged from the second connector segment 11B, and the second end surface 12c of the ring 12 will not cross the clamping groove 11c along the axial direction, and the clamping groove 11c is always within an area which is covered by the ring 12 along the axial direction. Similarly, when the ring 12 moves away from the blocking shoulder 11D, so as to enable the second end surface 12c of the ring 12 to contact with the top surface 200A of the medical device, the ring 12 will not be disengaged from the second connector segment 11B, and the first end surface 12b of the ring 12 will not cross the clamping groove 11c along the axial direction, and the clamping groove 11c is always within the area which is covered by the ring 12 along the axial direction.

That is to say, within the possible range of movement of the ring 12 along the axial direction, the clamping groove 11c is always located within the area which is covered by the ring 12 along the axial direction. This can prevent the ring 12 from being completely disengaged from the clamping groove 11c, and increase the probability of anti-disengagement of the securing element 15 by the ring 12.

It should be noted that in the case where the connection element 13 is connected with the second connector segment 11B (for example, including two cases: the second connection segment 133 mentioned above can be fixed to the inner pipe 112, or the second connection segment 133 can be separated from the inner pipe 112 and fall into the avoidance groove 111a), the ring 12 may not be in contact with the blocking shoulder 11D or with the top surface 200A of the connection arm 200.

For example, the outer diameter of the ring 12 is not greater than the outer diameter of the first connector segment 11A. For example, the outer diameter of the ring 12 is the same as that of the first connector segment 11A, and the outer wall of the ring 12 is approximately flush with the outer wall of the first connector segment 11A. For further example, the outer diameter of the ring 12 is less than the outer diameter of the first connector segment 11A, and the outer wall of the first connector segment 11A extends beyond the outer wall of the ring 12 in a radial direction.

In an embodiment provided with the protective sleeve 14, a size of the protective sleeve 14 can be designed based on the outer diameter of the first connector segment 11A, and the ring 12 hardly affects the shape and size of the protective sleeve 14.

Of course, in other embodiments, the outer wall of the ring 12 may also extend beyond the outer wall of the first connector segment 11A in a radial direction, that is, the outer diameter of the ring 12 may be greater than the outer diameter of the first connector segment 11A.

For example, a length of the ring 12 along its axial direction does not exceed a length of the second connector segment 11B. In this way, an end of the ring 12, which end is away from the blocking shoulder 11D, will never extend beyond the second connector segment 11B along the axial direction, thus reducing the impact of the ring 12 on the connection position of medical device.

For example, a length of the protective sleeve 14 along its axial direction is greater than a length of the ring 12 along its axial direction. When the protective sleeve 14 is at the installation position, both ends of the ring 12 along the axial direction of the ring 12 itself do not extend beyond opposite ends of the protective sleeve 14 along the axial direction of the protective sleeve 14 itself. That is to say, the protective sleeve 14 completely covers the ring 12, and when viewed from the outside of the support arm 100, the ring 12 cannot be seen.

An embodiment of this disclosure provides a support arm, including an arm body, a securing element 15 (refer to FIGS. 10 and 11), and a ring 12.

The specific type of medical device is not limited. For example, it could be a light head assembly of a surgical light.

The medical device is provided with a connection arm 200, which is connected with an insertion portion 201.

Please refer to FIG. 10. The connector 11 is provided inside with an insertion cavity 11a for inserting the insertion portion 201 of the medical device. Specifically, a lower end of the insertion cavity 11a is provided with an opening 11b, and the insertion portion 201 is inserted into the insertion cavity 11a through the opening 11b. Please refer to FIGS. 7 and 11, a side wall of connector 11 is provided with a clamping groove 11c.

The ring 12 is capable of preventing the securing element 15 from disengaging from the insertion portion 201. The ring 12 is movably sleeved around an outer circumference of the connector 11, so as to at least move between a first position and a second position.

When the ring 12 is at the first position, the insertion groove 12d is connected with the clamping groove 11c, so as to enable the securing element 15 to be inserted into the clamping groove 11c through the insertion groove 12d to engage with the insertion portion 201.

When the ring 12 is at the second position, the ring 12 at least partially covers the securing element 15, so as to prevent the securing element 15 from disengaging from the insertion portion 201.

When assembling the support arm 100 of this embodiment, the ring 12 is sleeved around an outer circumference of the connector 11, the insertion portion 201 is inserted into the insertion cavity 11a, the ring 12 is moved to the first position, so as to align the insertion groove 12d with the clamping groove 11c, and then the securing element 15 is inserted into the clamping groove 11c through the insertion groove 12d, and further inserted into the insertion portion 201. That is to say, a portion of the securing element 15 is located inside the insertion portion 201, and another portion of the securing element 15 is located inside the clamping groove 11c. Then, the ring 12 is moved, which causes the insertion groove 12d and the securing element 15 to be at least partially misaligned, so as to enable the ring 12 to at least partially cover the securing element 15, and to prevent the securing element 15 from disengaging from the insertion portion 201. Under the self-gravity of the medical device, the medical device is suspended on the securing element 15 through the insertion portion 201, and the securing element 15 bears at least part of the weight of the medical device.

It should be noted that the aforementioned movement includes translation and/or rotation.

When the ring 12 covers the securing element 15, the securing element 15 is not easily disengaged under the cover of the ring 12. Therefore, the securing element 15 is not easily disengaged from the insertion portion 201, and the medical device is not easily dropped, improving the reliability and safety of the medical device.

For example, referring to FIG. 10, an outer circumference of the insertion portion 201 is provided with a convex ring 200b or a ring groove for limiting position. When the securing element 15 abuts against a bottom of the convex ring 200b or is inserted into the ring groove, the insertion portion 201 is suspended on the securing element 15 through the convex ring 200b or the ring groove. In this way, it can facilitate the rotation of the insertion portion 201 inside the insertion cavity 11a, thereby driving the rotation of the medical device.

It should be noted that the remaining structure of the support arm in this embodiment can adopt the structure of the support arm in any of the above embodiments, and will not be repeated here.

An embodiment of this disclosure provides a surgical light, including a light head assembly and a support arm 100 of any embodiment of this disclosure.

The light head assembly includes a light holder, a connection arm 200, and multiple light sources which sources are arranged at the light holder. The light sources are configured to project illumination lights to a surgical area, and the light holder is connected with the connection arm 200.

Please refer to FIG. 4. The connection arm 200 is connected with an insertion portion 201, and an outer circumference of the insertion portion 201 is provided with a convex ring 200b or a ring groove for limiting position. The insertion portion 201 is inserted into the insertion cavity 11a, and when the securing element 15 abuts against a bottom of the convex ring 200b or is inserted into the ring groove, the light head assembly is suspended on the securing element 15 through the insertion portion 201, so as to enable the light head assembly to be rotatable relative to the connector 11. That is to say, the light head assembly is connected with the support arm 100 through the connection arm 200.

In the surgical light of this embodiment, the support arm 100 and the connection arm are connected with each other through a securing element 15, so that the light head assembly can be connected with the support arm 100 and rotate about the axis of the insertion portion 201. It should be noted that the type of surgical light is not limited, for example, it can be a shadowless light.

In the description of this disclosure, the reference to the terms "one embodiment", "some embodiments", "examples", "specific examples", or "some examples" means that the specific features, structures, materials, or characteristics described in conjunction with the embodiment or example are included in at least one embodiment or example of this disclosure. In this disclosure, the illustrative expressions of the above terms do not necessarily refer to the same embodiments or examples. Moreover, the specific features, structures, materials, or characteristics described can be combined in any one or more embodiments or examples in an appropriate manner. In addition, those skilled in this field can combine the different embodiments or examples described in this disclosure, as well as the features of different embodiments or examples, without conflicting with each other.

The above are only preferred embodiments of this disclosure and are not intended to limit this disclosure. For those skilled in the art, this disclosure can have various changes and modifications. Any modification, equivalent replacement, improvement, etc. made within the principle of this disclosure are included in the protection scope of this disclosure.

## Claims

1. A support arm (100) comprising:
an arm body, which is provided with a connector (11), wherein the connector is provided inside with an insertion cavity (11a) for inserting an insertion portion (201) of a medical device; wherein a side wall of the connector is provided with a clamping groove (11c);
a securing element (15);
a ring (12) that is capable of preventing the securing element from disengaging from the insertion portion, wherein the ring is provided with an insertion groove (12d), and movably sleeved around an outer circumference of the connector, so as to at least move between a first position and a second position;
when the ring is at the first position, the insertion groove is connected with the clamping groove, so as to enable the securing element to be inserted into the clamping groove through the insertion groove to engage with the insertion portion;
when the ring is at the second position, the ring is configured to at least partially cover the securing element, so as to prevent the securing element from disengaging from the insertion portion.

2. The support arm according to claim 1, **characterized in that**, further comprising a connection element (13), wherein the ring is connected with the connector through the connection element and is capable of being fixed at the second position through the connection element.

3. The support arm according to claim 2, **characterized in that**, the connector comprises a housing (111) and an inner pipe (112), the housing is sleeved around an outer circumference of the inner pipe, the clamping groove penetrates through a side wall of the housing and a side wall of the inner pipe; wherein the side wall of the housing is provided with an avoidance groove (111a), the ring is provided with a connection hole (12a), one end of the connection element penetrates through the connection hole and the avoidance groove; wherein when said end of the connection element is connected with the inner pipe, the connection element fixes the ring at the second position, when said end of the connection element is separated from the inner pipe, the ring is capable of driving the connection element to move along the avoidance groove.

4. The support arm according to claim 3, **characterized in that**, the connection element comprises a first connection segment (132) which is in a clearance fit with the connection hole, and a second connection segment (133) which is connected with the inner pipe, wherein the second connection segment is capable of being in threaded engagement with the connection hole, so as to penetrate through the connection hole; wherein a width of the avoidance groove is greater than a diameter of the second connection segment, and when the second connection segment is separated from the inner pipe, the second connection segment is accommodated inside the avoidance groove.

5. The support arm according to claim 3, **characterized in that**, the connection element comprises a first connection segment which is in a clearance fit with the connection hole, and a second connection segment which is connected with the inner pipe, wherein the second connection segment is capable of being in threaded engagement with the connection hole, so as to penetrate through the connection hole; wherein at least at a position which is adjacent to the first connection segment, a diameter of the second connection segment is greater than that of the first connection segment; a length of the first connection segment is greater than a depth of a corresponding portion of the connection hole; and a length of the second connection segment is less than a distance between an inner wall of the ring and an outer wall of the inner pipe, but greater than a distance between the inner wall of the ring and an outer wall of the housing.

6. The support arm according to claim 5, **characterized in that**, an aperture of the connection hole is greater than an outer diameter of the first connection segment; wherein a wall of the connection hole is provided with internal threads for threaded engagement with the second connection segment, when the second connection segment penetrates through the connection hole.

7. The support arm according to claim 3, **characterized in that**, a distance between an outer wall of the inner pipe and an outer wall of the ring is less than a length of the connection element; wherein when the connection element is separated from the inner pipe, the connection element protrudes from the outer wall of the ring; when the connection element fixes the ring at the second position, the connection element does not extend beyond the outer wall of the ring.

8. The support arm according to claim 3, **characterized in that**, the avoidance groove is an arc-shaped groove, which extends along a circumferential direction of the housing; or the avoidance groove is a curved groove, which extends along a circumferential direction and an axial direction of the housing; or the avoidance groove comprises multiple groove segments, which extend along a circumferential direction and an axial direction of the housing, and are connected with one another;
when the connection element is separated from the inner pipe, the connection element penetrates through and is arranged inside the avoidance groove, a fit between the avoidance groove and the connection element defines a movement trajectory of the ring.

9. The support arm according to claim 1, **characterized in that**, the connector comprises a first connector segment and a second connector segment, wherein an outer diameter of the second connector segment is less than an outer diameter of the first connector segment, a blocking shoulder is formed at a connection point between the first connector segment and the second connector segment, the ring surrounds an outer circumference of the second connector segment, an inner diameter of the ring is less than the outer diameter of the first connector segment, the clamping groove is arranged at the second connector segment;
wherein when the ring moves along its axial direction to a position where the ring is in contact with the blocking shoulder and/or to a position where the ring is in contact with the medical device, the ring at least partially covers the securing element; and/or
the medical device is provided with a top surface (200a) which faces the blocking shoulder, wherein the top surface is in contact with the ring, so as to block the ring from continuing to move towards the medical device along an axial direction of the ring; a distance between the clamping groove and the blocking shoulder, as well as a distance between the clamping groove and the top surface, are both less than a length of the ring along said axial direction.

10. The support arm according to claim 3, **characterized in that**, further comprising a protective sleeve (14), which is sleeved around the outer circumference of the connector and is capable of moving along an axial direction of the ring; wherein the protective sleeve has an installation position and an avoidance position; at the installation position, the protective sleeve is sleeved around an outer circumference of the ring and covers the insertion groove; at the avoidance position, the protective sleeve exposes the insertion groove;
when the connection element is separated from the inner pipe, a portion of the connection element protrudes from an outer wall of the ring, so as to limit an axial movement of the protective sleeve.

11. The support arm according to claim 10, **characterized in that**, a deformable snap-fit element (111A) is arranged on a circumferential outer surface of the connector;
a first positioning groove is arranged on an inner wall of the protective sleeve; when the protective sleeve is located at the installation position, a portion of the deformable snap-fit element is snapped into the first positioning groove, so as to limit the protective sleeve at the installation position at least along an axial direction of the protective sleeve; and/or
a second positioning groove is arranged on the inner wall of the protective sleeve; when the protective sleeve is located at the avoidance position, a portion of the deformable snap-fit element is snapped into the second positioning groove, so as to limit the protective sleeve at the avoidance position along the axial direction of the protective sleeve.

12. The support arm according to claim 9, **characterized in that**, the length of the ring along said axial direction does not exceed a length of the second connector segment; an outer diameter of the ring is not greater than the outer diameter of the first connector segment.

13. The support arm according to claim 1, **characterized in that**, further comprising a protective sleeve, which has an installation position; wherein at the installation position, the protective sleeve is sleeved around an outer circumference of the ring and covers the insertion groove; the protective sleeve is capable of moving upward along an axial direction of the ring to an avoidance position, at which position the insertion groove is exposed.

14. The support arm according to claim 13, **characterized in that**, a length of the protective sleeve along its axial direction is greater than a length of the ring along its axial direction; wherein when the protective sleeve is at the installation position, opposite ends of the ring along its axial direction do not extend beyond opposite ends of the protective sleeve along its axial direction.

15. A surgical light, **characterized in that**, comprising:
a light head assembly, which comprises a light holder, a connection arm (200), and multiple light sources which sources are arranged at the light holder; wherein the light sources are configured to project illumination lights to a surgical area, and the light holder is connected with the connection arm; and
the support arm according to any one of claims 1-14; wherein the connection arm is connected with the insertion portion, wherein an outer circumference of the insertion portion is provided with a convex ring or a ring groove for limiting position; when the securing element abuts against a bottom of the convex ring or is inserted into the ring groove, the light head assembly is suspended on the securing element through the insertion portion, so as to enable the light head assembly to be rotatable relative to the connector.

## Patentansprüche

1. Ein Tragarm (100), der Folgendes umfasst:
einen Armkörper, der mit einem Verbinder (11) versehen ist,
wobei der Verbinder im Inneren mit einem Einführhohlraum (11a) zum Einführen eines Einführabschnitts (201) einer medizinischen Vorrichtung versehen ist und eine Seitenwand des Verbinders mit einer Klemmnut (11c) versehen ist;
ein Sicherungselement (15);
einen Ring (12), der verhindern kann, dass sich das Sicherungselement vom Einführabschnitt löst, wobei der Ring mit einer Einführnut (12d) versehen ist und beweglich um einen Außenumfang des Verbinders geschoben ist, so dass er sich zumindest zwischen einer ersten Position und einer zweiten Position bewegen kann;
befindet sich der Ring in der ersten Position, ist die Einführnut mit der Klemmnut verbunden, sodass das Sicherungselement durch die Einführnut in die Klemmnut eingeführt werden kann, um in den Einführabschnitt einzugreifen;
befindet sich der Ring in der zweiten Position, ist der Ring so ausgebildet, dass er das Sicherungselement zumindest teilweise abdeckt, um zu verhindern, dass sich das Sicherungselement vom Einführabschnitt löst.

2. Der Tragarm gemäß Anspruch 1, **dadurch gekennzeichnet, dass** er ferner ein Verbindungselement (13) umfasst,
wobei der Ring über das Verbindungselement mit dem Verbinder verbunden ist und über das Verbindungselement in der zweiten Position fixiert werden kann.

3. Der Tragarm nach Anspruch 2, **dadurch gekennzeichnet, dass** der Verbinder ein Gehäuse (111) und ein Innenrohr (112) umfasst,
das Gehäuse um den Außenumfang des Innenrohrs geschoben ist und die Klemmnut eine Seitenwand des Gehäuses und eine Seitenwand des Innenrohrs durchdringt; wobei die Seitenwand des Gehäuses mit einer Ausweichnut (111a), der Ring mit einer Verbindungsbohrung (12a) versehen
Ist und ein Ende des Verbindungselements durch die Verbindungsbohrung und die Ausweichnut hindurchragt; wobei, wenn das genannte Ende des Verbindungselements mit dem Innenrohr verbunden ist, das Verbindungselement den Ring an der zweiten Position fixiert, und wenn das genannte Ende des Verbindungselements vom Innenrohr getrennt ist, der Ring in der Lage ist, das Verbindungselement dazu zu bringen, sich entlang der Ausweichnut zu bewegen.

4. Der Tragarm nach Anspruch 3, **dadurch gekennzeichnet, dass** das Verbindungselement ein erstes Verbindungssegment (132) umfasst, das in einer Spielpassung mit der Verbindungsbohrung steht, und ein zweites Verbindungssegment (133), das mit dem Innenrohr verbunden ist, wobei das zweite Verbindungssegment in Gewindeeingriff mit der Verbindungsbohrung gebracht werden kann, um durch die Verbindungsbohrung hindurchzudringen; wobei eine Breite der Ausweichnut größer ist als ein Durchmesser des zweiten Verbindungssegments, und wenn das zweite Verbindungssegment vom Innenrohr getrennt ist, das zweite Verbindungssegment innerhalb der Ausweichnut aufgenommen wird.

5. Der Tragarm nach Anspruch 3, **dadurch gekennzeichnet, dass** das Verbindungselement ein erstes Verbindungssegment umfasst, das in einer Spielpassung mit der Verbindungsbohrung steht, und ein zweites Verbindungssegment, das mit dem Innenrohr verbunden ist, wobei das zweite Verbindungssegment in Gewindeeingriff mit der Verbindungsbohrung gebracht werden kann, um durch die Verbindungsbohrung hindurchzudringen; wobei zumindest an einer Stelle, die an das erste Verbindungssegment angrenzt, der Durchmesser des zweiten Verbindungssegments größer ist als der des ersten Verbindungssegments; die Länge des ersten Verbindungssegments größer ist als die Tiefe eines entsprechenden Abschnitts der Verbindungsbohrung; und die Länge des zweiten Verbindungssegments kleiner ist als der Abstand zwischen einer Innenwand des Rings und einer Außenwand des Innenrohrs, jedoch größer als der Abstand zwischen der Innenwand des Rings und einer Außenwand des Gehäuses.

6. Der Tragarm nach Anspruch 5, **dadurch gekennzeichnet, dass** eine Öffnung der Verbindungsbohrung größer ist als ein Außendurchmesser des ersten Verbindungssegments; wobei eine Wand der Verbindungsbohrung mit einem Innengewinde versehen ist, um mit dem zweiten Verbindungssegment in Gewindeeingriff zu kommen, wenn das zweite Verbindungssegment die Verbindungsbohrung durchdringt.

7. Der Tragarm nach Anspruch 3, **dadurch gekennzeichnet, dass** ein Abstand zwischen einer Außenwand des Innenrohrs und einer Außenwand des Rings kleiner ist als eine Länge des Verbindungselements; wobei, wenn das Verbindungselement vom Innenrohr getrennt ist, das Verbindungselement aus der Außenwand des Rings herausragt und wenn das Verbindungselement den Ring an der zweiten Position fixiert, das Verbindungselement nicht über die Außenwand des Rings hinausragt.

8. Der Tragarm nach Anspruch 3, **dadurch gekennzeichnet, dass** die Ausweichnut eine bogenförmige Nut ist, die sich entlang einer Umfangsrichtung des Gehäuses erstreckt; oder dass die Ausweichnut eine gekrümmte Nut ist, die sich entlang einer Umfangsrichtung und einer axialen Richtung des Gehäuses erstreckt; oder dass die Ausweichnut mehrere Nutsegmente umfasst, die sich entlang einer Umfangsrichtung und einer axialen Richtung des Gehäuses erstrecken und miteinander verbunden sind; wenn das Verbindungselement vom Innenrohr getrennt ist, das Verbindungselement die Ausweichnut durchdringt und in dieser angeordnet ist, wobei eine Passung zwischen der Ausweichnut und dem Verbindungselement eine Bewegungsbahn des Rings definiert.

9. Der Tragarm nach Anspruch 1, **dadurch gekennzeichnet, dass** das Verbindungsstück ein erstes Verbindungsstücksegment und ein zweites Verbindungsstücksegment umfasst, wobei der Außendurchmesser des zweiten Verbindungsstücksegments kleiner ist als der Außendurchmesser des ersten Verbindungsstücksegments, an einer Verbindungsstelle zwischen dem ersten und dem zweiten Verbindungsstücksegment eine Sperrschulter ausgebildet ist, der Ring den Außenumfang des zweiten Verbindungsstücksegments umgibt, ein innerer Durchmesser des Rings kleiner ist als der Außendurchmesser des ersten Verbindungssegments und die Klemmnut am zweiten Verbindungssegment angeordnet ist
wobei, wenn sich der Ring entlang seiner axialen Richtung in eine Position bewegt, in der der Ring mit der Sperrschulter in Kontakt steht, und/oder in eine Position, in der der Ring mit dem medizinischen Gerät in Kontakt steht, der Ring das Sicherungselement zumindest teilweise abdeckt; und/oder
die medizinische Vorrichtung mit einer Oberseite (200a) versehen ist, die der Sperrschulter gegenüber liegt, wobei die Oberseite mit dem Ring in Kontakt steht, um den Ring daran zu hindern, sich entlang einer axialen Richtung des Rings weiter in Richtung der medizinischen Vorrichtung zu bewegen und ein Abstand zwischen der Klemmnut und der Sperrschulter sowie ein Abstand zwischen der Klemmnut und der Oberseite beide kleiner als eine Länge des Rings entlang der genannten axialen Richtung sind.

10. Der Tragarm nach Anspruch 3, **dadurch gekennzeichnet, dass** er ferner eine Schutzhülse (14) umfasst, die um den Außenumfang des Verbinders aufgeschoben ist und sich entlang einer axialen Richtung des Rings bewegen kann; wobei die Schutzhülse eine Einbauposition und eine Ausweichposition aufweist; in der Einbauposition die Schutzhülse um einen Außenumfang des Rings geschoben ist und die Einführnut bedeckt; in der Ausweichposition die Schutzhülse die Einführnut freigibt;
wenn das Verbindungselement vom Innenrohr getrennt ist, ein Teil des Verbindungselements aus einer Außenwand des Rings herausragt, um eine axiale Bewegung der Schutzhülse zu begrenzen.

11. Der Tragarm nach Anspruch 10, **dadurch gekennzeichnet, dass** ein verformbares Rastelement (111a) an einer Umfangsaußenfläche des Verbinders angeordnet ist;
eine erste Positionierungsnut an einer Innenwand der Schutzhülse angeordnet ist; wenn sich die Schutzhülse an der Einbauposition befindet, ein Abschnitt des verformbaren Rastelements in die erste Positionierungsnut einrastet, um die Schutzhülse in der Einbauposition zumindest entlang einer axialen Richtung der Schutzhülse zu begrenzen; und/oder
eine zweite Positionierungsnut an der Innenwand der Schutzhülse angeordnet ist; wenn sich die Schutzhülse in der Ausweichposition befindet, ein Abschnitt des verformbaren Rastelements in die zweite Positionierungsnut einrastet, um die Schutzhülse in der Ausweichposition entlang der axialen Richtung der Schutzhülse zu begrenzen.

12. Der Tragarm nach Anspruch 9, **dadurch gekennzeichnet, dass** die Länge des Rings entlang der genannten axialen Richtung eine Länge des zweiten Verbindungssegments nicht überschreitet und ein Außendurchmesser des Rings nicht größer ist als der Außendurchmesser des ersten Verbindungssegments.

13. Der Tragarm nach Anspruch 1, **dadurch gekennzeichnet, dass** er ferner eine Schutzhülse umfasst, die eine Einbauposition aufweist; wobei an der Einbauposition die Schutzhülse um einen Außenumfang des Rings geschoben ist und die Einführnut abdeckt und die Schutzhülse in der Lage ist, sich entlang einer axialen Richtung des Rings nach oben in eine Ausweichposition zu bewegen, in der die Einführnut freigelegt ist.

14. Der Tragarm nach Anspruch 13, **dadurch gekennzeichnet, dass** eine Länge der Schutzhülse entlang ihrer axialen Richtung größer ist als eine Länge des Rings entlang seiner axialen Richtung; wobei, wenn sich die Schutzhülse in der Einbaulage befindet, die gegenüberliegenden Enden des Rings entlang seiner axialen Richtung nicht über die gegenüberliegenden Enden der Schutzhülse entlang ihrer axialen Richtung hinausragen.

15. Eine Operationsleuchte, **dadurch gekennzeichnet, dass** sie Folgendes umfasst:
eine Leuchtenkopfbaugruppe, die einen Leuchtenhalter, einen Verbindungsarm (200) und mehrere Lichtquellen umfasst, die am Leuchtenhalter angeordnet sind; wobei die Lichtquellen so konfiguriert sind, dass sie Beleuchtungslicht auf einen Operationsbereich projizieren, und der Leuchtenhalter mit dem Verbindungsarm verbunden ist; und
der Tragarm gemäß einem der Ansprüche 1 bis 14; wobei der Verbindungsarm mit dem Einführabschnitt verbunden ist und ein Außenumfang des Einführabschnitts mit einem konvexen Ring oder einer Ringnut zur Positionsbegrenzung versehen ist; wenn das Befestigungselement an einer Unterseite des konvexen Rings anliegt oder in die Ringnut eingeführt ist und die Leuchtenkopfbaugruppe über den Einführabschnitt an dem Befestigungselement aufgehängt wird, so dass die Lichtkopfbaugruppe relativ zu dem Verbinder drehbar ist.

## Revendications

1. Bras de support (100) comprenant :
un corps de bras, lequel est doté d'un raccord (11), où le raccord est à l'intérieur pourvu d'une cavité d'insertion (11a) destinée à insérer une partie d'insertion (201) d'un dispositif médical ; où une paroi latérale du raccord est pourvue d'une rainure d'emboîtement (11c) ;
un élément de fixation (15) ;
une bague (12) qui est en mesure d'empêcher l'élément de fixation de se désengager de la partie d'insertion, où la bague est pourvue d'une rainure d'insertion (12d), et emmanchée de manière mobile autour d'une circonférence extérieure du raccord, de sorte à au moins se déplacer entre une première position et une seconde position ;
lorsque la bague se trouve à la première position, la rainure d'insertion est reliée à la rainure d'emboîtement, de sorte à permettre à l'élément de fixation d'être inséré dans la rainure d'emboîtement à travers la rainure d'insertion pour s'engager avec la partie d'insertion ;
lorsque la bague se trouve à la seconde position, la bague est configurée pour au moins en partie recouvrir l'élément de fixation, de sorte à empêcher l'élément de fixation de se désengager de la partie d'insertion.

2. Bras de support selon la revendication 1, **caractérisé en ce qu'**il comprend en outre un élément de raccordement (13), où la bague est reliée au raccord par le biais de l'élément de raccordement et peut être fixée à la seconde position par le biais de l'élément de raccordement.

3. Bras de support selon la revendication 2, **caractérisé en ce que** le raccord comprend un logement (111) et un tube intérieur (112), le logement est emmanché autour d'une circonférence extérieure du tube intérieur, la rainure d'emboîtement pénètre à travers une paroi latérale du logement et une paroi latérale du tube intérieur ; où la paroi latérale du logement est pourvue d'une rainure de dégagement (111a), la bague est pourvue d'un orifice de raccordement (12a),
une première extrémité de l'élément de raccordement pénètre à travers l'orifice de raccordement et la rainure de dégagement ; où lorsque ladite extrémité de l'élément de raccordement est reliée au tube intérieur, l'élément de raccordement fixe la bague à la seconde position, lorsque ladite extrémité de l'élément de raccordement est séparée du tube intérieur, la bague peut entraîner l'élément de raccordement pour le déplacer le long de la rainure de dégagement.

4. Bras de support selon la revendication 3, **caractérisé en ce que** l'élément de raccordement comprend un premier segment de raccordement (132), lequel est en ajustement avec jeu avec l'orifice de raccordement, et un second segment de raccordement (133) lequel est relié au tube intérieur, où le second segment de raccordement peut être en engagement vissé avec l'orifice de raccordement, de sorte à pénétrer à travers l'orifice de raccordement ; où une largeur de la rainure de dégagement est supérieure à un diamètre du second segment de raccordement, et lorsque le second segment de raccordement est séparé du tube intérieur, le second segment de raccordement est reçu au sein de la rainure de dégagement.

5. Bras de support selon la revendication 3, **caractérisé en ce que** l'élément de raccordement comprend un premier segment de raccordement lequel est en ajustement avec jeu avec l'orifice de raccordement, et un second segment de raccordement lequel est relié au tube intérieur, où le second segment de raccordement peut être en engagement vissé avec l'orifice de raccordement, de sorte à pénétrer à travers l'orifice de raccordement ; où au moins à une position qui est adjacente au premier segment de raccordement, un diamètre du second segment de raccordement est supérieur à celui du premier segment de raccordement ; une longueur du premier segment de raccordement est supérieure à une profondeur d'une partie correspondante de l'orifice de raccordement ; et une longueur du second segment de raccordement est inférieure à une distance entre une paroi intérieure de la bague et une paroi extérieure du tube intérieur, mais supérieure à une distance entre la paroi intérieure de la bague et une paroi extérieure du logement.

6. Bras de support selon la revendication 5, **caractérisé en ce qu'**une ouverture de l'orifice de raccordement est supérieure à un diamètre extérieur du premier segment de raccordement ; où une paroi de l'orifice de raccordement est pourvue de filets internes pour un engagement vissé avec le second segment de raccordement, lorsque le second segment de raccordement pénètre à travers l'orifice de raccordement.

7. Bras de support selon la revendication 3, **caractérisé en ce qu'**une distance entre une paroi extérieure du tube intérieur et une paroi extérieure de la bague est inférieure à une longueur de l'élément de raccordement ; où lorsque l'élément de raccordement est séparé du tube intérieur, l'élément de raccordement dépasse de la paroi extérieure de la bague ; lorsque l'élément de raccordement fixe la bague à la seconde position, l'élément de raccordement ne s'étend pas au-delà de la paroi extérieure de la bague.

8. Bras de support selon la revendication 3, **caractérisé en ce que** la rainure de dégagement est une rainure en forme d'arc, laquelle s'étend le long d'une direction circonférentielle du logement ; ou la rainure de dégagement est une rainure incurvée, laquelle s'étend le long d'une direction circonférentielle et d'une direction axiale du logement ; ou la rainure de dégagement comprend plusieurs segments de rainure, lesquels s'étendent le long d'une direction circonférentielle et d'une direction axiale du logement, et sont reliés les uns aux autres ;
lorsque l'élément de raccordement est séparé du tube intérieur, l'élément de raccordement pénètre à travers et est agencé à l'intérieur de la rainure de dégagement, un ajustement entre la rainure de dégagement et l'élément de raccordement définit une trajectoire de déplacement de la bague.

9. Bras de support selon la revendication 1, **caractérisé en ce que** le raccord comprend un premier segment de raccord et un second segment de raccord, où un diamètre extérieur du second segment de raccord est inférieur à un diamètre extérieur du premier segment de raccord, un épaulement de blocage est formé au niveau d'un point de liaison entre le premier segment de raccord et le second segment de raccord, la bague entoure une circonférence extérieure du second segment de raccord, un diamètre intérieur de la bague est inférieur au diamètre extérieur du premier segment de raccord, la rainure d'emboîtement est agencée au niveau du second segment de raccord ;
où lorsque la bague se déplace le long de sa direction axiale jusqu'à une position où la bague est en contact avec l'épaulement de blocage et/ou jusqu'à une position où la bague est en contact avec le dispositif médical, la bague recouvre au moins en partie l'élément de fixation ; et/ou
le dispositif médical est pourvu d'une surface supérieure (200a) laquelle fait face à l'épaulement de blocage, où la surface supérieure est en contact avec la bague, de sorte à bloquer la bague pour l'empêcher de se déplacer davantage vers le dispositif médical le long d'une direction axiale de la bague ; une distance entre la rainure d'emboîtement et l'épaulement de blocage, ainsi qu'une distance entre la rainure d'emboîtement et la surface supérieure, sont toutes deux inférieures à une longueur de la bague le long de ladite direction axiale.

10. Bras de support selon la revendication 3, **caractérisé en ce qu'**il comprend en outre un manchon de protection (14), lequel est emmanché autour de la circonférence extérieure du raccord et peut se déplacer dans une direction axiale de la bague ; où le manchon de protection présente une position d'installation et une position de dégagement ; à la position d'installation, le manchon de protection est emmanché autour d'une circonférence extérieure de la bague et recouvre la rainure d'insertion ; à la position de dégagement, le manchon de protection expose la rainure d'insertion ;
lorsque l'élément de raccordement est séparé du tube intérieur, une partie de l'élément de raccordement dépasse d'une paroi extérieure de la bague, de sorte à limiter un déplacement axial du manchon de protection.

11. Bras de support selon la revendication 10, **caractérisé en ce qu'**un élément d'emboîtement déformable (111A) est agencé sur une surface extérieure circonférentielle du raccord ;
un première rainure de positionnement est agencée sur une paroi intérieure du manchon de protection ; lorsque le manchon de protection est situé à la position d'installation, une partie de l'élément d'emboîtement déformable est emboîtée dans la première rainure de positionnement, de sorte à limiter le manchon de protection à la position d'installation au moins le long d'une direction axiale du manchon de protection ; et/ou
une seconde rainure de positionnement est agencée sur la paroi intérieure du manchon de protection ; lorsque le manchon de protection est situé à la position de dégagement, une partie de l'élément d'emboîtement déformable est emboitée dans la seconde rainure de positionnement, de sorte à limiter le manchon de protection à la position de dégagement le long de la direction axiale du manchon de protection.

12. Bras de support selon la revendication 9, **caractérisé en ce que** la longueur de la bague le long de ladite direction axiale ne dépasse pas une longueur du second segment de raccord ; un diamètre extérieur de la bague n'est pas supérieur au diamètre extérieur du premier segment de raccord.

13. Bras de support selon la revendication 1, **caractérisé en ce qu'**il comprend en outre un manchon de protection, lequel présente une position d'installation ; où à la position d'installation, le manchon de protection est emmanché autour d'une circonférence extérieure de la bague et recouvre la rainure d'insertion ; le manchon de protection peut se déplacer vers le haut le long d'une direction axiale de la bague jusqu'à une position de dégagement, à laquelle position la rainure d'insertion est exposée.

14. Bras de support selon la revendication 13, **caractérisé en ce qu'**une longueur du manchon de protection le long de sa direction axiale est supérieure à une longueur de la bague le long de sa direction axiale ; où lorsque le manchon de protection se trouve à la position d'installation, des extrémités opposées de la bague le long de sa direction axiale ne s'étendent pas au-delà d'extrémités opposées du manchon de protection le long de sa direction axiale.

15. Lampe chirurgicale, **caractérisée en ce qu'**elle comprend :
un ensemble de tête de lampe, lequel comprend un support de lampe, un bras de raccordement (200), et plusieurs sources de lumière lesquelles sources sont agencées au niveau du support de lampe ; où les sources de lumière sont configurées pour projeter des lumières d'éclairage vers une zone chirurgicale, et le support de lampe est relié au bras de raccordement ; et
le bras de support selon l'une quelconque des revendications 1 à 14 ; où le bras de raccordement est relié à la partie d'insertion, où une circonférence extérieure de la partie d'insertion est pourvue d'une bague convexe ou d'une rainure de bague destinée à la limiter une position ; lorsque l'élément de fixation bute contre un fond de la bague convexe ou est inséré dans la rainure de bague, l'ensemble de tête de lampe est suspendu sur l'élément de fixation par le biais de la partie d'insertion, de sorte à permettre à l'ensemble de tête de lampe de pouvoir tourner par rapport au raccord.
